(19) Europäisches Patentamt
European Patent Office
Office européen des brevets

(11) **EP 4 085 826 B1**

(12) **EUROPEAN PATENT SPECIFICATION**

(45) Date of publication and mention
of the grant of the patent:
**26.03.2025 Bulletin 2025/13**

(21) Application number: **21179558.8**

(22) Date of filing: **15.06.2021**

(51) International Patent Classification (IPC):
$A61B\ 5/024^{(2006.01)}$        $A61B\ 5/11^{(2006.01)}$
$A61B\ 5/00^{(2006.01)}$        $A61B\ 5/0205^{(2006.01)}$
$A61B\ 5/08^{(2006.01)}$        $A61B\ 5/113^{(2006.01)}$

(52) Cooperative Patent Classification (CPC):
**A61B 5/02438; A61B 5/0205; A61B 5/02444;
A61B 5/0816; A61B 5/1102; A61B 5/113;
A61B 5/486; A61B 5/6898; A61B 5/7221;
A61B 5/7257; A61B 5/7278;** A61B 2562/0219

(54) **SMARTPHONE HEART RATE AND BREATHING RATE DETERMINATION USING ACCURACY MEASUREMENT WEIGHTING**

SMARTPHONE-HERZFREQUENZ- UND ATEMFREQUENZBESTIMMUNG UNTER VERWENDUNG EINER GENAUIGKEITSMESSUNGSGEWICHTUNG

DÉTERMINATION DE LA FRÉQUENCE CARDIAQUE ET DE LA FRÉQUENCE RESPIRATOIRE SUR SMARTPHONE EN UTILISANT UNE PONDÉRATION DES MESURES DE PRÉCISION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.05.2021 US 202117307970**

(43) Date of publication of application:
**09.11.2022 Bulletin 2022/45**

(73) Proprietor: **Koa Health Digital Solutions S.L.U.**
**08018 Barcelona (ES)**

(72) Inventors:
• **Lucchesi, Federico**
**00174 Rome (IT)**
• **Maffei, Giovanni**
**08006 Barcelona (ES)**
• **Lantz, Johan**
**08019 Barcelona (ES)**

(74) Representative: **Casper, Catharina**
**Lorenz Seidler Gossel**
**Rechtsanwälte Patentanwälte**
**Partnerschaft mbB**
**Widenmayerstraße 23**
**80538 München (DE)**

(56) References cited:
**US-A1- 2016 278 647      US-A1- 2020 121 224**
**US-A1- 2020 260 962**

• **LAHDENOJA OLLI ET AL: "Atrial Fibrillation Detection via Accelerometer and Gyroscope of a Smartphone", IEEE JOURNAL OF BIOMEDICAL AND HEALTH INFORMATICS, IEEE, PISCATAWAY, NJ, USA, vol. 22, no. 1, 1 January 2018 (2018-01-01), pages 108 - 118, XP011675370, ISSN: 2168-2194, [retrieved on 20180102], DOI: 10.1109/JBHI.2017.2688473**

**Description**

TECHNICAL FIELD

The described embodiments relate generally to the determination of human heart rate and human breathing rate using smartphone accelerometers, and to related structures and methods.

BACKGROUND INFORMATION

There are many cellular telephone-based or smartphone-based heart rate monitors. All of these devices have shortcomings and problems. For example, many require the use of an additional sensor or wearable device. Some require the user to wear a finger monitor, a wrist strap, a watch, or to press on a sensor. The sensor might be a sensor that is connected to the smartphone via wires, or might be a wireless sensor. Some require the user to press a finger, or multiple fingers, onto the touchscreen of the smartphone. Some even attempt to detect physiological functions by processing images of the subject taken by the camera of the smartphone. In contrast to these systems, a easy-to-use, reliable, inexpensive and accurate smartphone-based heart rate and breathing rate monitoring system is desired that involves no sensors other than the accelerometers of a smartphone.

US10285650B2 (Heart monitoring device and method) describes a set of processing steps to filter the accelerometer signal of a hypothetical wearable device and to extract the estimated heart rate via ballistocardiography (i.e., the mechanical component of the pulsating blood in the body). Time-domain analysis of the signal (e.g., peak detection) is used, and the device does not extract breathing information. Breathing rate information is considered an artifact to be removed.

US10674424B2 (Method and apparatus for measuring physiological parameters) describes a set of distributed sensors comprising accelerometers and gyroscopes configured to infer heart rate, breathing rate and heart rate variability from the user's wrist and head. A computer-based method dynamically weights the contribution of each signal depending on user characteristics and use case. Weighting of monitored signals are not weighted over individual output values, and the method does not use intrinsic spectral properties of the signal.

US10617308B2 (Device and method for estimating the heart rate during motion) describes a portable device and method to estimate the heart rate of a user in motion. This is done by combining a standard ECG sensor (prone to motion artifacts) with a motion sensor. When the quality of the ECG reading drops below a certain threshold, a heart rate estimate is inferred from user motion (e.g., heart rate proportional to amount of motion). The method does not establish a threshold of accuracy below which the importance of a given sample is proportionally reduced. Rather, the method switches to use a different estimation method.

EP3318179A2 describes a method for measuring respiration rate and heart rate using dual camera of smartphone.

Biofeedback devices generally involve methods and interfaces to provide explicit feedback to the user about her physiological state by mapping physiological parameters to changes in the interface. For example, US20140316191A1 (Biofeedback Virtual Reality Sleep Assistant) describes a virtual reality system comprising physiological sensors and a head mounted display. The system maps the biosignal for sleep tracking (e.g., heart rate, breathing rate) to images on the screen with the goal of dynamically favoring sleep. The system does not use audio signals to guide the user during a breathing exercise for relaxation (with the goal of reducing stress), nor does it use biofeedback to display the performance of the user on the screen (e.g., how well the user followed the breathing rhythm).

WO2006113900A2 (Methods and devices for relieving stress) describes a portable device equipped with a PPG sensor able to measure respiration patterns of the user. The breathing wave pattern is displayed to the user on a screen in real time to help follow a breathing pattern with the goal of reducing stress. The method displays waveforms and not determined rates, and does so in real time and not at the end of an exercise, nor are both breathing and heart information displayed.

The conference paper entitled "Beat-to-Beat Heart Rate Detection By Smartphone's Accelerometers: Validation With ECG," by Federica Landreani et al., Proceedings of the Annual International Conference of the IEEE Engineering in Medicine and Biology Society (2016), considers the possibility and the feasibility of measuring beat-to-beat heart cycle during using peak detection time domain signal processing of the x, y and z orthogonal axis seismocardiographic (SCG) accelerometric signals from an Apple iPhone6 smartphone in order to determine heart rate. Fiducial point detection is used as described in the paper entitled "Heart Rate Variability Analysis Using A Seismocardiogram Signal," by J. Ramos-Castro et al., Conference Proceedings of the Annual International Conference of the IEEE Engineering in Medicine and Biology Society, pp. 5642-5645 (2012). Offline processing on a remote computer employs noise-sensitive peak detection for the detection of heart rate. The method does not involve accuracy estimation for adjustment of detected heart rate values, nor does it involve the determination of breathing rate. The offline peak-detection method is so sensitive to noise that the method can only work well in the absence of noise and cannot be effectively employed in real time entirely on a typical smartphone. Further relevant prior art is US2020/260962A1 and a publication by Lahdenoja Olli et al: Atrial Fibrillation Detection via Accelerometer and Gyroscope of a Smartphone", IEEE Journal of Biomedical and Health Informatics, January 2018.

SUMMARY

A smartphone exercise app and an associated heart rate (HR) and breathing rate (BR) plugin provide biofeedback to a smartphone user in the context of a smartphone-based relaxation exercise to reduce mental stress. The system employs a processing pipeline that combines inference biosignals (for example, heart rate and breathing rate) from the 3-axis accelerometer of the smartphone with an accuracy estimation method that enhances robustness of the estimate. More specifically, predetermined spectral entropy to weight mapping information is stored on the smartphone. This information is stored on the smartphone before the user uses the smartphone exercise app to determine heart rate and breathing rate. There is one such mapping for each of a plurality of use cases. For example, there is a first mapping for a use case when a heart rate (as opposed to breathing rate) is to be detected, when a user holding the smartphone is in a sitting position (as opposed to the smartphone resting on the user's chest when the user is in the supine position), and when the smartphone is detected to a first type of smartphone (as opposed to a second type of smartphone). There is a second mapping for a use case when breathing rate is to be detected, when the user is holding the smartphone in the sitting position, and when the smartphone is detected to be of the first type.

The exercise app starts and uses the 3-axis accelerometer of the smartphone to generate a plurality of accelerometer output data sets. Each accelerometer output data set contains a sequence of accelerometer output samples that are captured by the accelerometer over a period of time (for example, over a 20.48 second period of time). Each accelerometer output sample includes an x-axis accelerometer output value, a y-axis accelerometer output value, a z-axis accelerometer output value, and a timestamp. In one example, accelerometer output samples are received from the accelerometer at a rate of one hundred output samples per second. In one example, one accelerometer output data set of 2048 output samples is generated each two seconds, with the output samples in the data set representing data captured over a 20.48 second period of time.

From each set of x-axis, y-axis and z-axis accelerometer output data sets, a single ballistocardiogram (BCG) data set is generated. Frequency domain processing is performed on the single BCG data set thereby generating an estimated heart rate (EHR) value. Accordingly, for each successive accelerometer output data set a corresponding estimated heart rate (EHR) value is generated.

Next, from each successive accelerometer output data set, a corresponding estimated accuracy (EA) value is determined, thereby determining a sequence of EA values. An EA value is determined using: 1) a measure of the power spectral entropy of the output samples in the accelerometer output data set, as well as 2) the appropriate spectral entropy to weight mapping previously stored on the smartphone. From the EA value, an EHR weight value is determined. Accordingly, there is one such EHR weight value determined for each EHR value.

The EHR weight values are then used to adjust the sequence of EHR values, thereby generating a sequence of adjusted heart rate (AHR) values. This use of EHR weights allows EHR values that are estimated to be less accurate to have less effect on the AHR values than HER values that are estimated to be more accurate. Accuracy of the AHR values as compared to actual heart rate is improved by use of the spectral entropy to weight mapping information and the determined estimated accuracy values of the EHR values.

In addition to determining heart rate, an estimated breathing rate (EBR) value is determined for each accelerometer output data set. Frequency domain signal processing is performed on each of the x-axis accelerometer output data set, the y-axis accelerometer output data set, and the z-axis accelerometer output data set. In one example, the EBR value is determined to be the frequency of the Fast Fourier Transform (FFT) component having the maximum amplitude of the most periodic of the x-axis, y-axis and z-axis data sets.

Next, for each successive accelerometer output data set, a corresponding estimated accuracy (EA) value is determined, thereby determining a sequence of EA values. An EA value is determined using: 1) a measure of the power spectral entropy of the data in the accelerometer output data set, as well as 2) the spectral entropy to weight mapping information previously stored on the smartphone. From the EA value, a weight value is determined. Accordingly, there is one such weight value determined for each EBR value. The weight values are then used to adjust the sequence of EBR values, thereby generating a sequence of adjusted heart rate (ABR) values. Accuracy of the ABR values as compared to actual breathing rate is improved by use of the spectral entropy to weight mapping information and the determined estimated accuracy values of the EBR values.

The determination of the EHR and EBR values occurs entirely on the smartphone, without any of the user's potentially sensitive biosignal information being transmitted or communicated from the smartphone to another device. The only sensor involved is the smartphone's 3-axis accelerometer, and the user does not have to wear or interact with any external additional sensor or measurement device. The user needs merely to hold the smartphone in the user's hand, or to place the smartphone on the user's chest when lying in a face-upward supine position.

In one embodiment, the AHR and ABR values determined by the plugin is used by the exercise app to provide customized feedback to the user o how the user is doing in the overall relaxation exercise. For example, the exercise app causes breathing rhythm prompts to be communicated to the user based on the user's real-time detected heart rate and/or breathing rate. After a period of time of the user's using the exercise app, final results of the relaxation exercise are displayed to the user on the smartphone touchscreen and the final results are logged on the smartphone for future reference.

Further details and embodiments and methods and techniques are described in the detailed description below. This summary does not purport to define the invention. The invention is defined by the claims.

BRIEF DESCRIPTION OF THE DRAWINGS

The accompanying drawings, where like numerals indicate like components, illustrate embodiments of the invention.

FIG. 1 is a simplified diagram of software executing on a smartphone in system in accordance with one embodiment of the present invention.

FIG. 2 is a flowchart of an exemplary operation of the smartphone of FIG. 1.

FIG. 3 is a diagram of the smartphone of FIG. 1 when the exercise app is causing an instruction to be displayed to the user.

FIG. 4 is a diagram of the user when the user is sitting in the proper sitting position using the smartphone of FIG. 1.

FIG. 5 is a diagram of one possible way the breathing rate results and AHR results may be displayed to the user on the touchscreen of the smartphone of FIG. 1.

FIG. 6 is a more detailed diagram of the timeline graph portion of what is displayed on the touchscreen as shown in FIG. 5.

FIG. 7 is a diagram of the results of the relaxation exercise being displayed on the touchscreen of the smartphone of FIG. 1.

FIG. 8 is a graph showing a relationship between normalized spectral entropy and accuracy, for the use case of heart rate being determined, the user being in the sitting position, and the smartphone having an iOS operating system.

DETAILED DESCRIPTION

Reference will now be made in detail to some embodiments of the invention, examples of which are illustrated in the accompanying drawings.

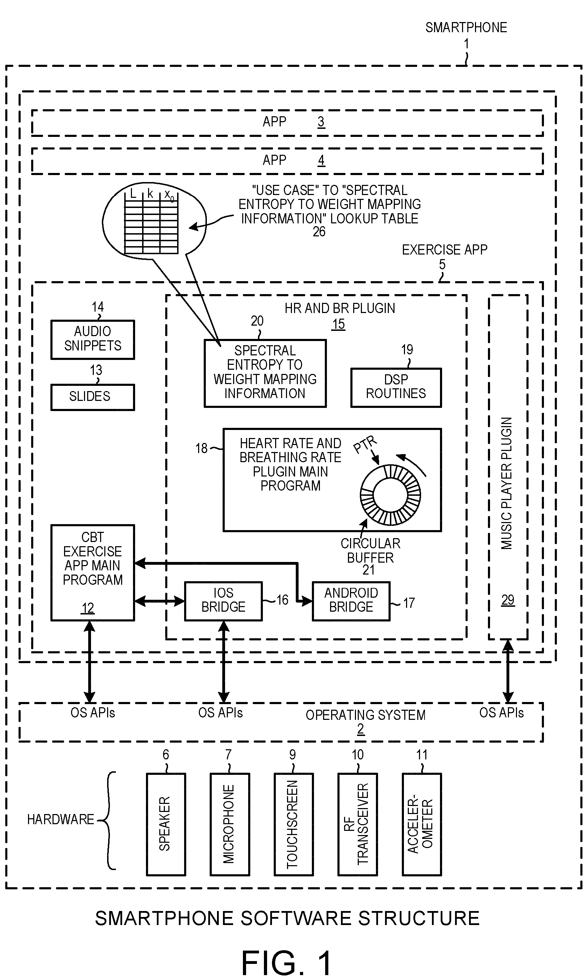

FIG. 1 is a simplified diagram of software executing on a smartphone 1 in accordance with one embodiment of the present invention. A smartphone is a mobile cellular telephone that performs many of the functions of a computer, typically having a touchscreen interface, internet access, and an operating system capable of running downloaded mobile applications ("apps"). In the present example, the smartphone is an iPhone6 cellular telephone available from Apple Computer of Cupertino, California. Executing on the iOS operating system 2 are a plurality of applications or "apps" 3-4 and a novel exercise app 5 in accordance with an embodiment of the present invention. The hardware of the cellular telephone includes, among other parts not illustrated, a speaker 6, a microphone 7, a touchscreen 9, RF cellular telephone transceiver electronics 10, and an accelerometer 11. The exercise app 5 includes an app main program 12, multiple files of data that can be presented to a user 22 on the display of the touchscreen as slides 13, multiple audio snippets 14 that can be played for the user 22 by the speaker, a heart rate (HR) and breathing rate (BR) plugin 15, and a music player plugin 29. The HR and BR plugin 15 in turn includes an iOS bridge portion 16, an android bridge portion 17, a heart rate and breathing rate plugin main program portion 18, digital signal processing (DSP) routines portion 19, and spectral entropy to weight mapping information 20. All of the code of exercise app 5 including the music player plugin 29 is compiled together and is present on the smartphone 1 in the form of one bundle of executable code.

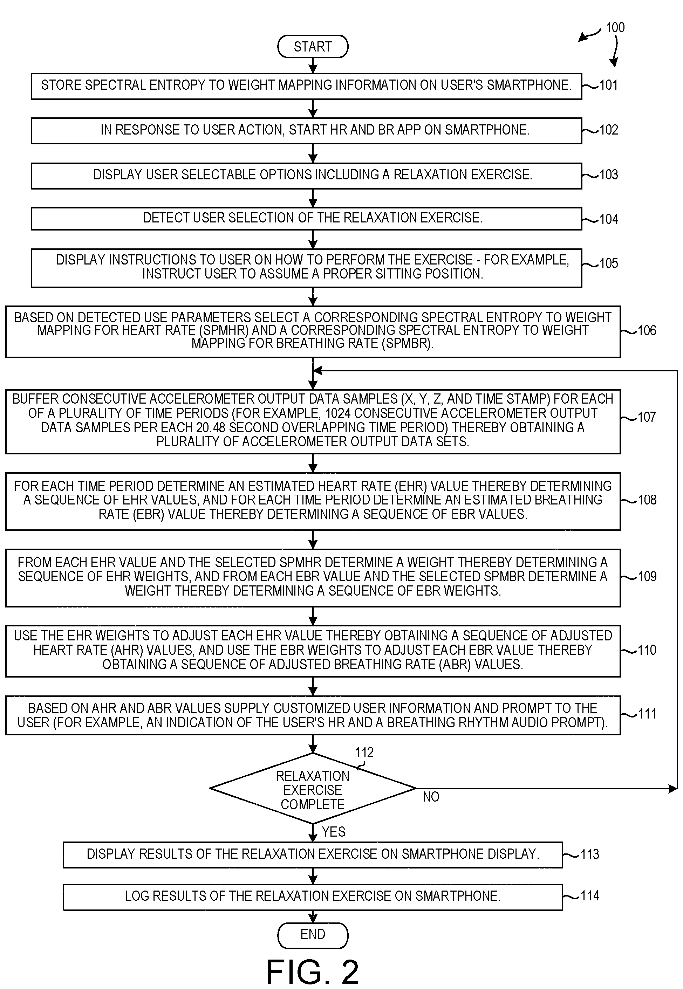

FIG. 2 is a flowchart 100 of one exemplary operation of the smartphone 1 of FIG. 1. Stored on the smartphone (step 101) is a set of spectral entropy to weight mappings 20. In the present example, there are eight such mappings that map an input normalized spectral entropy value to an output weight value. This information is represented by block 20 in FIG. 1.

In response to the user 22 pressing an icon on the touchscreen of the smartphone, the exercise app 5 starts (step 102) which in turns starts the HR and BR plugin 15. The plugin 15 via the iOS bridge 16 makes a request of the operating system 2 (via an iOS function call "startaccelerometerupdatestoqueue") to start a subscription to send accelerometer data back to the plugin. The plugin 15 receives the accelerometer data through the iOS bridge 16 at a rate of one hundred accelerometer samples per second. Each accelerometer sample includes an accelerometer x-axis data value, an accelerometer y-axis data value, an accelerometer z-axis data value, and a time stamp. The accelerometer data values are double precision floating point numbers, and as collected data range from a low value of 0.0 g to a high value of about 7.7 g, and have a resolution of approximately 0.018 g. The heart rate and breathing rate plugin main program 18 receives the incoming stream of accelerometer output samples via the iOS bridge 16 and pushes them sequentially into a circular buffer data structure 21. The circular buffer 21 has 2048 buffer storage locations, each of which can store one accelerometer output sample, where an accelerometer output sample includes an x-axis accelerometer value, a y-axis accelerometer value, a z-axis accelerometer value, and a timestamp. As accelerometer output samples are stored into the circular buffer 21, a head pointer PTR is incremented so that the head pointer PTR always points to the last filled buffer storage location. A tail pointer need not be maintained because the number of buffer slots in the circular buffer (2048 buffer storage locations) is known.

Next, the exercise app main program 12 consults the program structure to determine which of the slides will be shown next and proceeds by making an internal function call to present the correct slide 13 (step 103) to the user 22 on the smartphone touchscreen 9 along with a selectable set of options. One of these user selectable options is a relaxation exercise. In the present example, the user 22 presses on the touchscreen selection for the relaxation exercise. The exercise app 5 gets a callback from the operating system 2 indicating to the exercise app 5 that the user has pressed the touchscreen to make this selection. This causes the slide implementation handler 13 to send a slide selected event to the exercise app main program 12 (step 104) thereby alerting the exercise main program 12 of the user's selection of the relaxation exercise. In response to the user's selection of the relaxation exercise, the exercise app main program 12 makes an internal function call to cause another of the slides 13 to be presented (step 105) to the user 2 on the smartphone touchscreen 9 instructing the user to assume a proper sitting position for the relaxation exercise to be performed.

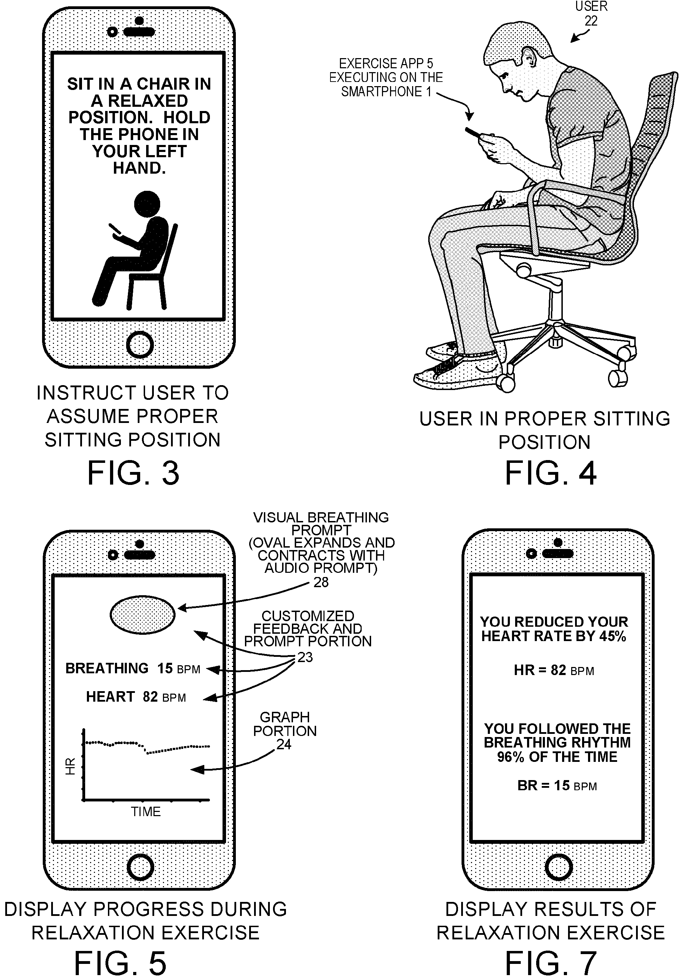

FIG. 3 is a diagram of the smartphone 1 when the exercise app 5 is causing the instruction to be displayed to the user.

Next, in response to the instruction, the user 22 assumes the proper sitting position. FIG. 4 is a diagram of the user 22 when the user is sitting in the proper sitting position.

Next, the HR and BR plugin 15 selects one of the eight stored spectral entropy to weight mappings 20 (step 106) to be the spectral entropy to weight mapping for heart rate (SPMHR) to be used in the selected relaxation exercise. Which spectral entropy to weight mapping is selected is based on detected use parameters. In the present example, a first use parameter is whether heart rate is to be determined or whether breathing rate is to be determined. A second use parameter is whether the user will be performing the exercise in the sitting position with the user sitting on a chair (with the smartphone in the user's left hand), or in the supine position (user lying on the user's back, face upward, with the smartphone lying on the user's chest). A third use parameter is whether the smartphone is of a first type (for example, an iPhone type) or a second type (for example, an android type). For this parameter, all smartphones are classified as belonging to one of these two general groups. There are eight possible combinations of these three use binary parameters, so eight spectral entropy to weight mappings are stored. One of these mappings is selected to be the spectral entropy to weight mappings for heart rate (SPMHR). In addition, another of the stored spectral entropy to weight mappings is selected (step 106) to be the spectral entropy to weight mapping for breathing rate (SPMBR). Which spectral entropy to weight mapping is selected to be the SPMBR is based on the detected use parameters.

Accelerometer output samples are being stored into the circular buffer 21 at a continuous rate of one hundred accelerometer output samples per second, and the circular buffer 21 at any given time stores the last 2048 accelerometer output samples. The heart rate and breathing rate plugin main program 18, however, reads out the present circular buffer 21 contents (2048 output samples) each two minutes. One such set of output sample values is referred to as an "accelerometer output data set". Because the circular buffer stores 2048 accelerometer output samples which represents accelerometer output samples collected over one 20.48 second time period, only 200 of the accelerometer output samples in each successive accelerometer output data set are newly stored accelerometer output samples. The time periods corresponding to successive accelerometer output data sets overlap one another by 1848 samples. The accelerometer output data sets of the stream of accelerometer output data sets that are output in this way are downsampled by two (every other accelerometer output sample is discarded) so that each accelerometer output data set consists of 1024 accelerometer output samples. In this way, the following are generated (step 107): 1) a stream of x-axis accelerometer output data sets where each x-axis accelerometer output data set is 1024 x-axis accelerometer output data values, 2) a stream of y-axis accelerometer output data sets where each y-axis accelerometer output data set is 1024 y-axis accelerometer output data values, 3) a set of z-axis accelerometer output data sets is generated where each z-axis accelerometer output data set is 1024 z-axis accelerometer output data values, and 4) a stream of timestamps.

For the time period of each "accelerometer output data set", the heart rate and breathing rate plugin main program 18 determines (step 108) an estimated heart rate (EHR) value, thereby determining a sequence of EHR values. Also, for each time period of each "accelerometer output data set", the heart rate and breathing rate plugin main program 18 determines (step 108) an estimated breathing rate (EBR) value, thereby determining a sequence of EBR values.

Determination of an EHR value: The heart rate and breathing rate plugin main program 18 determines one EHR value from each corresponding "accelerometer output data set". From each of the x-axis accelerometer output data values of the accelerometer output data set, the y-axis accelerometer output data values of the accelerometer output data set, and the z-axis accelerometer output data values of the accelerometer output data set, a time-domain moving average is determined (e.g., with a window size corresponding to a duration of about 0.1 seconds). As the values of this time domain moving average are determined, each successive time domain average is subtracted from the corresponding accelerometer output data value. This reduces the non-stationarity of the accelerometer output data values.

A Butterworth low-pass filter (e.g., with cut-off frequency of 7.0Hz, and order 1) is applied to each of the three resulting streams of values (three "signals"). The Butterworth low-pass filter is applied again to the reversed version of each resulting signal.

For each of the resulting three signals, the mean determined. From each signal value the mean is subtracted, and each resulting value is divided by its standard deviation. This results in a sequence of modified x-axis values, a sequence of modified y-axis values, and a sequence of modified z-axis values.

The L2 norm of each associated set of three data values (an x-axis value of the modified x-axis values, a y-axis value of the modified y-axis values, and a z-axis value of the modified z-axis values) is determined. There are 1024 such sets. The L2 norm is determined by taking the square root of the sum of the square of the three associated values. This results in a single sequence of 1024 values known as a BCG (or, ballistocardiogram).

A Butterworth band-pass filter (e.g., with low and high cut-off frequencies of 0.6Hz and 2.7Hz respectively, and an order of one) is then applied to this BCG, and is applied again to the reversed resulting signal. A Fast Fourier Transform (FFT) is applied to the resulting signal. A moving average is applied to the resulting transform outputs (i.e., a moving average (with a window of five) of the magnitudes of the frequency components in the frequency domain). The frequency component of the FFT output that has the maximum amplitude is determined. The frequency of this component is determined to be the estimated heart rate (EHR) value.

Determination of an EBR value: The heart rate and breathing rate plugin main program 18 determines one EBR value from each corresponding 1024-value "accelerometer output data set". From each of the x-axis accelerometer output data values of the accelerometer output data set, the y-axis accelerometer output data values of the accelerometer output data set, and the z-axis accelerometer output data values of the accelerometer output data set, the following is done. The mean is subtracted from each output data value, and the result is divided by its standard deviation. A Butterworth band-pass filter (e.g., with low and high cut-off frequencies of 0.07Hz and 1.6Hz respectively, and order one) is applied, and is applied again to the reversed resulting signal. A Fast Fourier Transform (FFT) is performed on the resulting signal, thereby generating a set of frequency components each having an amplitude. This process is performed for each of the x-axis accelerometer output data values, the y-axis accelerometer output data values, and the z-axis output data values, thereby generating three resulting FFT output signals. The most periodic one of these three signals is defined to be the signal (either the x-axis derived signal, the y-axis derived signal, or the z-axis derived signal) that has the frequency component of the greatest value. The frequency corresponding to this frequency component of the greatest value is determined to be the estimated breathing rate (EBR) value.

In the manner described above, both an EHR value and an EBR value are determined for the time period of each "accelerometer output data set", thereby generating sequence of EHR values and a sequence of EBR values (step 107).

For each EHR value, the heart rate and breathing rate plugin main program 18 determines one EHR weight value (step 108) from the corresponding "accelerometer output data set". The power spectral entropy (PSE) is then computed, and the PSE is computed differently for HR and BR purposes. In the case of HR detection, the power spectral entropy of the BSG signal is determined, whereas in the case of BR the PSE of the most periodic signal amount the three axes is determined. In the present example where heart rate is being determined, the power spectrum $X(w_i)$ of the signal is determined first, using a Fast Fourier Transform (FFT) for discrete frequencies $w_i$ between zero and half of the sampling frequency. The normalized Power Spectral Density $P(w_i)$ is given by equation (1) below:

$$P(\omega_i) = \frac{1}{N}\left|X(\omega_i)\right|^2$$

where N is the number of frequency bins. $P(\omega_i)$ is then mapped to a probability density function p according to equation (2) below:

$$p_i = \frac{P(\omega_i)}{\sum_j P(\omega_j)}$$

The PSE value is given by equation (3) below:

$$PSE = -\sum_{i=1}^{n} p_i \ln p_i$$

A normalized PSE value is determined by dividing the PSE value by ln(N). Once the normalized PSE value is determined, an estimated accuracy (EA) value is determined according to equation (4) below:

$$EA = \frac{L}{1 + e^{-k(x-x_0)}}$$

where the input variable x is the normalized PSE value. The parameters L, k and $x_0$ are constants that define a relationship between an input value of x (the determined normalized PSE value) and the output estimated accuracy EA value being determined. The set of the three parameters L, k and $x_0$ is determined from lookup table 26 from the input detected use parameters mentioned in connection with step 106. Note the illustration of the lookup table 26 in FIG. 1. As mentioned above in connection with step 106, there are three input detected use parameters: 1) the first use parameter is whether heart rate is to be determined or whether breathing rate is to be determined, 2) the second use parameter is whether the user will be performing the exercise in the sitting position or the supine position, and 3) the third use parameter is whether the smartphone is of a first type (iPhone smartphone type) or a second type (android smartphone type). For each of the eight combinations of these three input parameters, the lookup table 26 in block 20 of FIG. 1 stores a corresponding set of the three parameters L, k and $x_0$. The three parameters L, k and $x_0$ define a relationship between the input value (the normalized PSE value) and the output value EA (the estimated accuracy EA value). Accordingly, from the normalized PSE value an estimated accuracy EA value is determined in accordance with equation (4) above. Then, from the estimated accuracy EA value, the weight value w(EA) is determined according to equation (5) below:

$$w(EA) = \frac{EA^m}{t^{m-1}} \text{ if } 0 \leq EA < t, \text{ whereas } w(EA) = EA \text{ if } t \leq EA \leq 1$$

where t is an accuracy threshold parameter, and m is a below-threshold decay parameter. There is one such EHR weight value w(EA) determined (step 109) for each separate EHR value. Importantly, the relation of equation (5) allows more importance to be given to estimated EHR values that have higher measured confidence. The same process is repeated using each EBR value so that one EBR weight value w(EA) is determined (step 109) for each EBR value.

Next, the EHR weights are used in a moving window to adjust each consecutive EHR value to obtain an adjusted heart rate (AHR) value (step 110). The five EHR weights within a moving window of five weights are L-1 normalized to determine five normalized weights. Each normalized weight within the window is multiplied by its corresponding EHR value, and the five resulting products are summed in order to determine one adjusted EHR value for the window. The window is then shifted five weight values in time, and this is how the downsampling with a stride of ten seconds is accomplished. The five EHR weights within the new window are L-1 normalized to determine another five normalized weights, and those five normalized weights are used to determine another adjust EHR value. This process is repeated as the window is shifted through all the 1024 EHR values so that an adjusted AHR value is determined for each of the original EHR values. In the same way that the EHR weights are used to adjust the corresponding 1024 EHR values to obtain 1024 AHR values, the same process is repeated using EBR weights to adjust the 1024 EBR values in order to obtain 1024 ABR values (step 110).

The heart rate and breathing rate plugin main program 18 then downsamples (with a stride of ten seconds) the sequence of 1024 AHR values so there is one AHR value generated each ten seconds. Likewise, the sequence of 1024 BR values is downsampled (with a stride of ten seconds) so there is one ABR value generated each ten seconds.

The heart rate and breathing rate plugin main program 18 then assigns a Boolean accuracy value to each downsampled AHR value. In the present example, the Boolean accuracy value will be positive (True) if the percentage of confidence values, within the relative moving window, is greater than a certain accuracy threshold percentage (for example, 33%). The Boolean accuracy value will be negative (False) otherwise.

The CBT exercise app main program 12 sends a request to the plugin main program 18 via the iOS bridge 16 instructing the plugin main program 18 to return the AHR and ABR results. The CBT exercise app main program 12 receives the AHR and ABR results and causes the AHR and ABR results to be presented (step 111) to the user 22 via the touchscreen of the smartphone.

FIG. 5 is a diagram of one possible way the AHR results may be displayed to the user. What is displayed in this example includes a customized feedback and prompt portion 23 and a timeline graph portion 24. The customized feedback and prompt portion 23 includes a visual breathing prompt icon 28, an indication of the current determined breathing rate in breaths per minute, and an indication of the current determined heart rate in beats per minute. The visual breathing prompt icon 28 expands and contracts in concert with an audio breathing rhythm prompt. The audio breathing prompt is an audio snippet, the playback rate of which is timed and controlled by the CBT exercise app main program 12. The timeline graph portion 24 graphs heart rate (AHR) with respect to time as illustrated in FIG. 5. Accordingly, the exercise app 5 provides real time feedback to the user 22 about the user's physiological state.

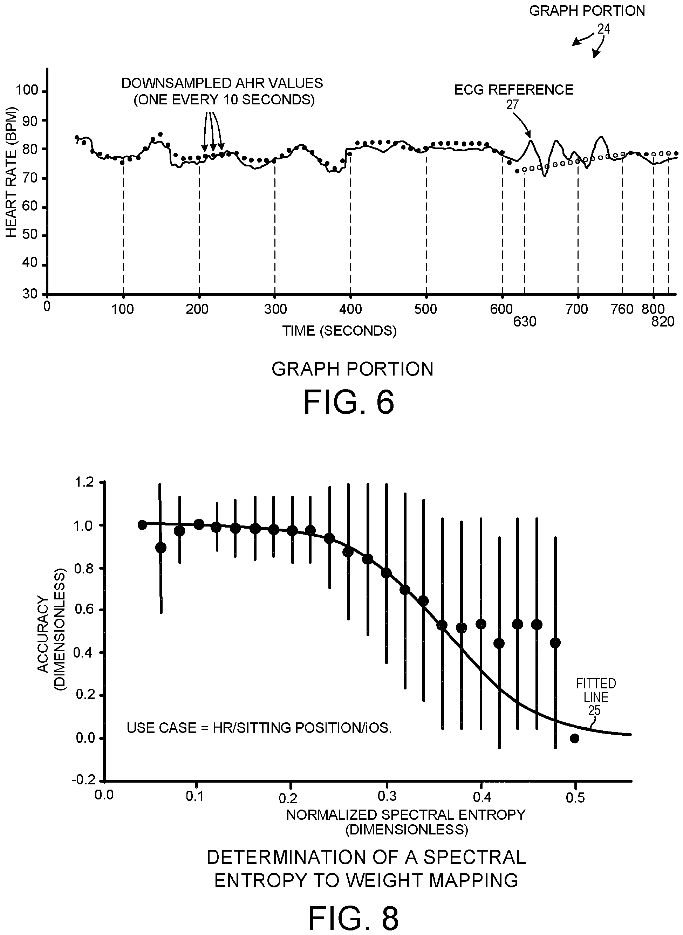

FIG. 6 is a more detailed diagram of the timeline graph portion 24 of FIG. 5. In the graph, the dots represent individual adjusted AHR values. There is one such AHR value each ten seconds. If the Boolean accuracy value of the AHR is true then the dot is displayed in the color green, whereas if the Boolean accuracy value of the AHR is false then the dot is displayed in the color orange. In the black and white patent document illustration of FIG. 6, a green dot is represented by a solid black dot, whereas an orange dot is represented by a black circle.

In the event that there are more than a predetermined number (for example two) of consecutive negative downsampled AHR values with a negative Boolean accuracy (Boolean accuracy value is False), then the AHR values are not displayed, but rather these negative accuracy measures are replaced with values determined by linear interpolation between the nearest positive Boolean accuracy AHR values (Boolean accuracy value is True) to the immediate left and immediate right of the consecutive negative values. These AHR dots are, however, still displayed in orange. For example, in FIG. 6, between time 630 seconds and time 760 seconds the fourteen AHR values have been replaced with interpolated values but the dots are still displayed in the orange color. If the number of consecutive AHR values that have negative Boolean accuracy values (False) is small (for example, two), then the AHR values are simply displayed as being of positive Boolean accuracy. In the case of FIG. 6, two such consecutive dots would be displayed in the color green. The line 27 is not shown on the smartphone touchscreen. The line 27 represents an electrocardiogram (ECG) reference signal, and is presented on FIG. 6 in this patent document just for reference to show how close the graphed AHR values are to the reference.

The CBT exercise app main program 12 calls a function in the music player plugin 29 to play one of the breathing rhythm audio prompt snippets of the audio snippets 14. The audio snippet is played with a controlled rhythm so as to lead the user 22 to breath in a more relaxed fashion.

If it is decided that the relaxation exercise is not yet complete (step 112), then processing proceeds back to step 107 to process another 20.48 seconds worth of accelerometer output samples. If it is decided that the relaxation exercise is complete (step 112), then the CBT exercise app main program 12 causes results of the relaxation exercise to the displayed (step 113) on the smartphone display.

FIG. 7 is a diagram of the results of the relaxation exercise being displayed on the touchscreen 9 of the smartphone 1. The results of the relaxation exercise are also logged (step 114) in memory on the smartphone. Importantly for privacy concerns, reliable HR and BR values are determined entirely on the smartphone without any collected physiological data being communicated out of the smartphone.

Determination of the Spectral Entropy to Weight Mappings: The spectral entropy to weight mappings referred to in step 101 of the flowchart of FIG. 2 are determined by collecting accelerometer output sample data from multiple subjects for each one of the eight permutations of the HR/BR parameter, the sitting/supine parameter, and the iOS/android parameter. While the accelerometer output sample data is collected, the subject is simultaneously monitored using an ECG device and a respiration rate monitor.

FIG. 8 illustrates accelerometer output sample data collected for the HR/sitting/iOS use case. The horizontal axis indicates the normalized spectral entropy. The vertical axis indicates accuracy. Each dot in the diagram represents the average of hundreds of EHR values, where the 1024 accelerometer sample output values (of an accelerometer output data set) from which an EHR value is determined have the normalized power spectral entropy as indicated on the horizontal axis in the graph. The accuracy of an EHR value is determined by comparing the EHR value to its corresponding ECG-generated value. In the graph of FIG. 8, the vertical bar passing through one of the dots represents both the maximum and minimum accuracies of the EHR values represented by the dot. After the data of the graph is collected, the parameter constants $L$, $k$ and $x_0$ are adjusted so that the equation of the function of equation (4) is heuristically fitted to the collected data. Reference numeral 25 identifies the fitted line for the example of FIG. 8. The spectral entropy to weight mapping that is stored in table 26 of FIG. 1 is given by the determined values of the three parameter constants $L$, $k$ and $x_0$. The same process is carried for breathing rate, with the subjects being monitored using the respiration rate monitor. After the breathing rate data is collected, the parameters $L$, $k$ and $x_0$ are adjusted to that the equation of the function of equation (4) is heuristically fitted to the collected breathing rate data.

Accuracy Of The AHR and ABR Determinations: A cardiograph is a device that generates electrocardiograms. An electrocardiogram (ECG) is a graph of voltage versus time of the electrical activity of the heart using electrodes placed on the skin of a subject around the heart. These electrodes detect the small electrical changes that are a consequence of cardiac muscle depolarization followed by repolarization during each heartbeat. There are three main components to an ECG: the P wave, which represents the depolarization of the atria; the QRS complex, which represents the rapid depolarization of the right and left ventricles; and the T wave, which represents the repolarization of the ventricles. The inter-beat interval (RR) is the time elapsed between the beginnings of two successive R-waves. This RR time interval is converted into a heart beats per second (HR) value. For purposes of determining the accuracy of the AHR values, the heart rate value determined from the ECG device is considered the gold standard.

Accuracy of the AHR as determined by the method of the flowchart of FIG. 2 is determined by monitoring a user with an ECG device while simultaneously using the smartphone 1 of FIG. 1 to generate AHR values in accordance with the method of FIG. 2. The mean absolute error between each AHR value and the corresponding heart rate value obtained from the ECG device is determined. To compute the accuracy of the heart rate determination of the method of FIG. 2 (the AHR values), a threshold of acceptable error is used (5 BPM for HR and 3 BPM for BR). The percentage of "close enough to the ground truth value" is determined, so this quantity is dimensionless. The accuracy gains of AHR and ABR with respect to EHR and EBR are about twenty-five to thirty percent. The absolute values of the accuracies depend substantially on the single data run, the smartphone model used, and the user position. It would therefore make more sense to talk about the accuracy gain, which is more general and can be expressed more concisely. But the accuracy computed on the overall data collected in absolute terms, EHR has an overall accuracy of about 0.73, and AHR has an overall accuracy of about 0.94. The accuracy gain is therefore (0.94-0.73)/(0.73*100)=28.7 percent. The data used in this determination was taken over a period of about thirty minutes for each of twenty-three users.

A respiration rate monitor is a device used to measure the breathing rate of a subject. One type of suitable respiration rate monitor, which allows monitoring the respiration rate, is a piezoelectric transducer (PZT) respiration sensor, that measures displacement variations induced by inhaling or exhaling. A PZT respiration sensor model includes the Biosignalsplux model PZT01102020, 1 Hertz bandwidth, 106 dB common mode rejection ratio (CMRR), 16-bit channel, sensor available from Plux-Wireless Biosignals, S.A., of Lisbon, Portugal. The data generated is measured as "displacement value in percentage of full scale". For purposes of determining the accuracy of the ABR values, the breathing rate value determined from the respiration rate monitor is considered the gold standard. Accuracy of the ABR as determined by the method of the flowchart of FIG. 2 is determined by monitoring a user with a respiration rate monitor while simultaneously using the smartphone 1 of FIG. 1 to generate ABR values in accordance with the method of FIG. 2. The mean absolute error between each ABR value and the corresponding breathing rate value obtained from the respiration rate monitor is determined. The accuracy gain of the breathing rate determination of the system of the method of FIG. 2 (the ABR values) is about twenty-five to thirty percent. In absolute terms, the ABR accuracy is about 0.92.

Although certain specific embodiments are described above for instructional purposes, the teachings of this patent document have general applicability and are not limited to the specific embodiments described above. Accordingly, various modifications, adaptations, and combinations of various features of the described embodiments can be practiced without departing from the scope of the invention as set forth in the claims.

**Claims**

1. A method, comprising:

(a) using one or more accelerometers of a smartphone to generate a plurality of accelerometer output data sets, wherein each accelerometer output data set contains a plurality of accelerometer output data values for a different period of time;
(b) determining from each accelerometer output data set a corresponding estimated heart rate, EHR, value, thereby determining a sequence of EHR values;
(c) determining from each accelerometer output data set a corresponding estimated accuracy, EA, value, thereby determining a sequence of EA values; and
**characterised by**
(d) using the sequence of EA values determined in (c) to adjust the sequence of EHR values determined in (b) thereby generating a corresponding sequence of adjusted heart rate, AHR, values, wherein steps (a), (b), (c) and (d) are performed by the smartphone.

2. The method of claim 1, wherein the adjusting of (d) involves determining, for each estimated accuracy, EA, value, a corresponding weight value, and wherein the generating of one of the AHR values of (d) involves determining a normalized weight value for each weight value of a sequence of the determined weight values and further involves using each of the normalized weight values to weight its corresponding EHR value in a weighted running average determination such that an output of the weighted running average determination is the AHR value.

3. The method of claim 1, wherein the determining in (c) of an estimated accuracy, EA, value from an accelerometer output data set involves determining a power spectral entropy value from the output data values of the accelerometer output data set, or wherein successive ones of the periods of time in (a) partially overlap one another in time.

4. The method of claim 1, further comprising:
(e) displaying on the smartphone a representation of at least one of the AHR values generated in (d).

5. The method of claim 1, further comprising:

(e) for each AHR generated in (d) determining whether the AHR value has an accuracy characteristic; and
(f) displaying on the smartphone a representation of each AHR value of a sequence of the AHR values generated in (d), wherein the representation of each AHR value of the sequence is displayed along with a visual indication of whether the AHR value has the accuracy characteristic, wherein preferably the representation of each AHR value is a graphical object rendered on a screen of the smartphone, and wherein the visual indication of whether the AHR value has the accuracy characteristic is indicated by a color of the graphical object.

6. The method of claim 1, wherein the one or more accelerometers of the smartphone include an x-axis accelerometer, a y-axis accelerometer and a z-axis accelerometer, and wherein each accelerometer output data set generated in (a) includes accelerometer output data values that are generated by the x-axis accelerometer, accelerometer output data values that are generated by the y-axis accelerometer, and accelerometer output data values that are generated by the z-axis accelerometer.

7. The method of claim 1, further comprising:
(e) storing spectral entropy to weight mapping information on the smartphone, and wherein the determining of (c) involves determining a power spectral entropy, PSE, value for each accelerometer output data set, and then for each accelerometer output data set using the PSE value of the accelerometer output data set and the stored spectral entropy to weight mapping information to generate the estimated accuracy, EA, value of the accelerometer output data set.

8. A method, comprising:

(a) storing spectral entropy to weight mapping information on a smartphone;
(b) using an accelerometer of the smartphone to generate an accelerometer output data set, wherein each accelerometer output data set contains a plurality of accelerometer output data values for a period of time;
(c) determining from the accelerometer output data set an estimated heart rate, EHR, value;
(d) determining from the accelerometer output data set a power spectral entropy, PSE, value; and
(e) using the PSE value determined in (d) and the spectral entropy to weight mapping information stored in (a) to adjust the EHR value thereby generating an adjusted heart rate, AHR, value, wherein steps (a) through (e) are performed by the smartphone.

9. The method of claim 10, wherein the spectral entropy to weight mapping information stored in (a) defines a relationship between a normalized version of the PSE value and an estimated accuracy, EA, value, wherein the EA value determines a weight value for the accelerometer output data set, and wherein (e) involves using the weight value for the accelerometer output data set to adjust the EHR value or wherein the spectral entropy to weight mapping information stored in (a) involves both (1) a first spectral entropy to weight mapping associated with a first use case of the smartphone and (2) a second spectral entropy to weight mapping associated with a second use case of the smartphone.

10. A method, comprising:

(a) storing first spectral entropy to weight mapping information on a smartphone;
(b) storing second spectral entropy to weight mapping information on the smartphone;
(c) using an accelerometer of the smartphone to generate an accelerometer output data set, wherein the accelerometer output data set contains a plurality of accelerometer output data values for a period of time;
(d) determining from the accelerometer output data set an estimated heart rate EHR, value;
(e) determining from the accelerometer output data set an estimated breathing rate, EBR, value;
(f) determining from the accelerometer output data set power spectral entropy, PSE, information;
(g) using the PSE information determined in (f) and the first spectral entropy to weight mapping information stored in (a) to adjust the EHR value thereby generating an adjusted heart rate, AHR, value; and

(h) using the PSE information determined in (f) and the second spectral entropy to weight mapping information stored in (b) to adjust the EBR value thereby generating an adjusted breathing rate, ABR, value, wherein steps (a) through (h) are performed by the smartphone.

11. The method of claim 10, wherein (c) involves generating a plurality of accelerator output data sets, wherein (d) involves determining an EHR value for each of the accelerator output data sets thereby determining a sequence of EHR values, wherein (g) involves using the first spectral entropy to weight mapping information to generate a weight value for each of the EHR values so that each EHR value has a corresponding weight value, and wherein the generating of the AHR value in (g) involves determining a weighted running average value from a plurality of the EHR values and their corresponding weight values, wherein preferably each accelerometer output data set contains a plurality of accelerometer output data values for a different period of time, and wherein successive ones of the different periods of time overlap one another in time.

12. The method of claim 10, wherein a plurality of spectral entropy to weight mappings is stored on the smartphone, wherein the first spectral entropy to weight mapping information of (a) is one of the spectral entropy to weight mappings of the plurality of spectral entropy to weight mappings, and wherein the second spectral entropy to weight mapping information of (b) is another of the spectral entropy to weight mappings of the plurality of spectral entropy to weight mappings.

13. The method of claim 10, wherein the method further comprising:

(i) the smartphone detecting a use case and based at least in part on the detected use case the smartphone selects one of the spectral entropy to weight mappings to be used in the adjusting of the EHR value in (g).

14. The method of claim 10, wherein the AHR value generated in (g) is one of a sequence of AHR values generated by the smartphone, wherein some of the AHR values of the sequence are displayed graphically on a screen of the smartphone in a first color, and wherein others of the AHR values of the sequence are displayed graphically on the screen of the smartphone in a second color.

15. The method of claim 10, wherein the AHR value generated in (g) is one of a sequence of AHR values generated by the smartphone, wherein the smartphone determines that some of the AHR values of the sequence are relatively more accurate whereas the smartphone determines that others of the AHR values of the sequence are relatively less accurate, wherein AHR values that are determined by the smartphone to be relatively more accurate are displayed graphically on a screen of the smartphone in a first color, and wherein AHR values that are determined by the smartphone to be relatively less accurate are displayed graphically on the screen of the smartphone in a second color or wherein the PSE information determined in (f) includes a first PSE value and a second PSE value, wherein the first PSE value is used in the generating of the AHR value in (g), and wherein the second PSE value is used in the generating of the ABR value in (h).

**Patentansprüche**

1. Verfahren, umfassend:

(a) Verwenden eines oder mehrerer Beschleunigungsmesser eines Smartphones zum Erstellen einer Vielzahl von Beschleunigungsmesserausgangsdatensätzen, wobei jeder Beschleunigungsmesserausgangsdatensatz eine Vielzahl von Beschleunigungsmesserausgangsdatenwerten für einen unterschiedlichen Zeitraum enthält;
(b) Bestimmen anhand von jedem Beschleunigungsmesserausgangsdatensatz eines entsprechenden geschätzten Herzfrequenzwerts (EHR-Werts), wodurch eine Sequenz von EHR-Werten bestimmt wird;
(c) Bestimmen anhand von jedem Beschleunigungsmesserausgangsdatensatz eines entsprechenden geschätzten Genauigkeitswerts (EA-Werts), wodurch eine Sequenz von EA-Werten bestimmt wird; und
**gekennzeichnet durch**
(d) Verwenden der in (c) bestimmten Sequenz von EA-Werten, um die in (b) bestimmte Sequenz von EHR-Werten anzupassen, wodurch eine entsprechende Sequenz von angepassten Herzfrequenzwerten (AHR-Werten) erstellt wird, wobei die Schritte (a), (b), (c) und (d) von dem Smartphone durchgeführt werden.

2. Verfahren nach Anspruch 1, wobei das Anpassen von (d) das Bestimmen, für jeden geschätzten Genauigkeitswert (EA-Wert), eines entsprechenden Gewichtungswerts beinhaltet, und wobei das Erstellen von einem der AHR-Werte von (d) das Bestimmen eines normalisierten Gewichtungswerts für jeden Gewichtungswert einer Sequenz der bestimmten Gewichtungswerte beinhaltet und ferner das Verwenden jedes der normalisierten Gewichtungswerte zum Gewichten seines entsprechenden EHR-Werts bei einer gewichteten gleitenden Durchschnittsbestimmung beinhaltet, so dass eine Ausgabe der gewichteten gleitenden Durchschnittsbestimmung der AHR-Wert ist.

3. Verfahren nach Anspruch 1, wobei das Bestimmen in (c) eines geschätzten Genauigkeitswerts (EA-Werts) anhand eines Beschleunigungsmesserausgangsdatensatzes das Bestimmen eines Leistungsspektralentropiewerts anhand der Ausgangsdatenwerte des Beschleunigungsmesserausgangsdatensatzes beinhaltet, oder wobei aufeinanderfolgende der Zeiträume in (a) sich bezüglich der Zeit teilweise einander überlappen.

4. Verfahren nach Anspruch 1, ferner umfassend:
(e) Anzeigen auf dem Smartphone einer Darstellung mindestens eines der in (d) erstellten AHR-Werte.

5. Verfahren nach Anspruch 1, ferner umfassend:

(e) für jede in (d) erstellte AHR, Bestimmen, ob der AHR-Wert ein Genauigkeitsmerkmal aufweist; und
(f) Anzeigen auf dem Smartphone einer Darstellung jedes AHR-Werts einer in (d) erstellten Sequenz der AHR-Werte, wobei die Darstellung jedes AHR-Werts der Sequenz zusammen mit einer visuellen Anzeige dahingehend, ob der AHR-Wert das Genauigkeitsmerkmal aufweist, angezeigt wird, wobei vorzugsweise die Darstellung jedes AHR-Werts ein grafisches Objekt ist, das auf einem Bildschirm des Smartphones gerendert wird, und wobei die visuelle Anzeige dahingehend, ob der AHR-Wert das Genauigkeitsmerkmal aufweist, durch eine Farbe des grafischen Objekts angegeben wird.

6. Verfahren nach Anspruch 1, wobei der eine oder die mehreren Beschleunigungsmesser des Smartphones einen X-Achsen-Beschleunigungsmesser, einen Y-Achsen-Beschleunigungsmesser und einen Z-Achsen-Beschleunigungsmesser einschließen, und wobei jeder in (a) erstellte Beschleunigungsmesserausgangsdatensatz Beschleunigungsmesserausgangsdatenwerte, die durch den X-Achsen-Beschleunigungsmesser erstellt werden, Beschleunigungsmesserausgangsdatenwerte, die durch den Y-Achsen-Beschleunigungsmesser erstellt werden, und Beschleunigungsmesserausgangsdatenwerte, die durch den Z-Achsen-Beschleunigungsmesser erstellt werden, einschließt.

7. Verfahren nach Anspruch 1, ferner umfassend:
(e) Speichern von Spektralentropie-Gewicht-Zuordnungsinformationen auf dem Smartphone, und wobei das Bestimmen von (c) das Bestimmen eines Leistungsspektralentropiewerts (PSE-Werts) für jeden Beschleunigungsmesserausgangsdatensatz und dann für jeden Beschleunigungsmesserausgangsdatensatz das Verwenden des PSE-Werts des Beschleunigungsmesserausgangsdatensatzes und der gespeicherten Spektralentropie-Gewicht-Zuordnungsinformationen zum Erstellen des geschätzten Genauigkeitswerts (EA-Werts) des Beschleunigungsmesserausgangsdatensatzes beinhaltet.

8. Verfahren, umfassend:

(a) Speichern von Spektralentropie-Gewicht-Zuordnungsinformationen auf einem Smartphone;
(b) Verwenden eines Beschleunigungsmessers des Smartphones zum Erstellen eines Beschleunigungsmesserausgangsdatensatzes, wobei jeder Beschleunigungsmesserausgangsdatensatz eine Vielzahl von Beschleunigungsmesserausgangsdatenwerten für einen Zeitraum enthält;
(c) Bestimmen anhand des Beschleunigungsmesserausgangsdatensatzes eines geschätzten Herzfrequenzwerts (EHR-Werts);
(d) Bestimmen anhand des Beschleunigungsmesserausgangsdatensatzes eines Leistungsspektralentropiewerts (PSE-Werts); und
(e) Verwenden des in (d) bestimmten PSE-Werts und der Spektralentropie zum Gewichten von in (a) gespeicherten Zuordnungsinformationen, um den EHR-Wert anzupassen, wodurch ein angepasster Herzfrequenzwert (AHR-Wert) erstellt wird, wobei die Schritte (a) bis (e) von dem Smartphone durchgeführt werden.

9. Verfahren nach Anspruch 10, wobei die in (a) gespeicherten Spektralentropie-Gewicht-Zuordnungsinformationen ein Verhältnis zwischen einer normalisierten Version des PSE-Werts und einem geschätzten Genauigkeitswert (EA-Wert) definiert, wobei der EA-Wert einen Gewichtungswert für den Beschleunigungsmesserausgangsdatensatz bestimmt, und wobei (e) das Verwenden des Gewichtungswerts für den Beschleunigungsmesserausgangsdatensatz zum Anpassen des EHR-Werts beinhaltet, oder wobei die in (a) gespeicherten Spektralentropie-Gewicht-Zuordnungsinformationen sowohl (1) eine erste Spektralentropie-Gewicht-Zuordnung in Verbindung mit einem ersten Anwendungsfall des Smartphones als auch (2) eine zweite Spektralentropie-Gewicht-Zuordnung in Verbindung mit einem zweiten Anwendungsfall des Smartphones beinhaltet.

10. Verfahren, umfassend:

(a) Speichern von ersten Spektralentropie-Gewicht-Zuordnungsinformationen auf einem Smartphone;
(b) Speichern von zweiten Spektralentropie-Gewicht-Zuordnungsinformationen auf dem Smartphone;
(c) Verwenden eines Beschleunigungsmessers des Smartphones zum Erstellen eines Beschleunigungsmesserausgangsdatensatzes, wobei der Beschleunigungsmesserausgangsdatensatz eine Vielzahl von Beschleunigungsmesserausgangsdatenwerten für einen Zeitraum enthält;
(d) Bestimmen anhand des Beschleunigungsmesserausgangsdatensatzes eines geschätzten Herzfrequenzwerts (EHR-Werts);
(e) Bestimmen anhand des Beschleunigungsmesserausgangsdatensatzes eines geschätzten Atemfrequenzwerts (EBR-Werts);
(f) Bestimmen anhand des Beschleunigungsmesserausgangsdatensatzes von Leistungsspektralentropieinformationen (PSE-Informationen);
(g) Verwenden der in (f) bestimmten PSE-Informationen und der in (a) gespeicherten ersten Spektralentropie-Gewicht-Zuordnungsinformationen, um den EHR-Wert anzupassen, wodurch ein angepasster Herzfrequenzwert (AHR-Wert) erstellt wird; und
(h) Verwenden der in (f) bestimmten PSE-Informationen und der in (b) gespeicherten zweiten Spektralentropie-Gewicht-Zuordnungsinformationen, um den EBR-Wert anzupassen, wodurch ein angepasster Atemfrequenzwert (ABR-Wert) erstellt wird, wobei die Schritte (a) bis (h) von dem Smartphone durchgeführt werden.

11. Verfahren nach Anspruch 10, wobei (c) das Erstellen einer Vielzahl von Beschleunigungsmesserausgangsdatensätzen beinhaltet, wobei (d) das Bestimmen eines EHR-Werts für jeden der Beschleunigungsmesserausgangsdatensätze beinhaltet, wodurch eine Sequenz von EHR-Werten bestimmt wird, wobei (g) das Verwenden der ersten Spektralentropie-Gewicht-Zuordnungsinformationen zum Erstellen eines Gewichtungswerts für jeden der EHR-Werte beinhaltet, so dass jeder EHR-Wert einen entsprechenden Gewichtungswert aufweist, und wobei das Erstellen des AHR-Werts in (g) das Bestimmen eines gewichteten gleitenden Durchschnittswerts anhand einer Vielzahl der EHR-Werte und ihrer entsprechenden Gewichtungswerte beinhaltet, wobei vorzugsweise jeder Beschleunigungsmesserausgangsdatensatz eine Vielzahl von Beschleunigungsmesserausgangsdatenwerten für einen unterschiedlichen Zeitraum enthält, und wobei aufeinanderfolgende der unterschiedlichen Zeiträume sich bezüglich der Zeit einander überlappen.

12. Verfahren nach Anspruch 10, wobei eine Vielzahl von Spektralentropie-Gewicht-Zuordnungen auf dem Smartphone gespeichert werden, wobei die erste Spektralentropie-Gewicht-Zuordnungsinformation von (a) eine der Spektralentropie-Gewicht-Zuordnungen der Vielzahl von Spektralentropie-Gewicht-Zuordnungen ist, und wobei die zweite Spektralentropie-Gewicht-Zuordnungsinformation von (b) eine andere der Spektralentropie-Gewicht-Zuordnungen der Vielzahl von Spektralentropie-Gewicht-Zuordnungen ist.

13. Verfahren nach Anspruch 10, wobei das Verfahren ferner Folgendes umfasst:

(i) Erkennen durch das Smartphone eines Anwendungsfalls und Auswählen, durch das Smartphone, zumindest teilweise basierend auf dem erkannten Anwendungsfall, einer der Spektralentropie zum Gewichten von Zuordnungen, die bei dem Anpassen des EHR-Werts in (g) verwendet werden soll.

14. Verfahren nach Anspruch 10, wobei der in (g) erstellte AHR-Wert einer einer Sequenz von AHR-Werten ist, die von dem Smartphone erstellt werden, wobei einige der AHR-Werte der Sequenz grafisch auf einem Bildschirm des Smartphones in einer ersten Farbe angezeigt werden, und wobei andere der AHR-Werte der Sequenz grafisch auf dem Bildschirm des Smartphones in einer zweiten Farbe angezeigt werden.

15. Verfahren nach Anspruch 10, wobei der in (g) erstellte AHR-Wert einer einer Sequenz von AHR-Werten ist, die von dem Smartphone erstellt werden, wobei das Smartphone bestimmt, dass einige der AHR-Werte der Sequenz relativ genauer sind, während das Smartphone bestimmt, dass andere der AHR-Werte der Sequenz relativ ungenauer sind, wobei AHR-Werte, in Bezug auf die das Smartphone bestimmt, dass sie relativ genauer sind, grafisch auf einem Bildschirm des Smartphones in einer ersten Farbe angezeigt werden, und wobei AHR-Werte, in Bezug auf die das Smartphone bestimmt, dass sie relativ ungenauer sind, grafisch auf dem Bildschirm des Smartphones in einer zweiten Farbe angezeigt werden, oder wobei die in (f) bestimmten PSE-Informationen einen ersten PSE-Wert und einen zweiten PSE-Wert einschließen, wobei der erste PSE-Wert bei dem Erstellen des AHR-Werts in (g) verwendet wird, und wobei der zweite PSE-Wert bei dem Erstellen des ABR-Werts in (h) verwendet wird.

## Revendications

1. Procédé comprenant

(a) l'utilisation d'un ou de plusieurs accéléromètres d'un téléphone intelligent pour générer une pluralité d'ensembles de données de sortie d'accéléromètre, dans lequel chaque ensemble de données de sortie d'accéléromètre contient une pluralité de valeurs de données de sortie d'accéléromètre pour une période de temps différente ;

(b) la détermination à partir de chaque ensemble de données de sortie d'accéléromètre d'une valeur EHR [estimated heart rate value - valeur de fréquence cardiaque estimée] correspondante, en déterminant ainsi une séquence de valeurs EHR ;

(c) la détermination à partir de chaque ensemble de données de sortie d'accéléromètre d'une valeur EA [estimated accuracy value - valeur de précision estimée] correspondante, en déterminant ainsi une séquence de valeurs EA ; et

**caractérisé par**

(d) l'utilisation de la séquence de valeurs EA déterminée dans l'étape (c) pour ajuster la séquence de valeurs EHR déterminée dans l'étape (b) en générant ainsi une séquence correspondante de valeurs AHR [adjusted heart rate value - valeur de fréquence cardiaque ajustée], dans lequel les étapes (a), (b), (c) et (d) sont effectuées par le téléphone intelligent.

2. Procédé selon la revendication 1, dans lequel l'ajustement de l'étape (d) implique la détermination, pour chaque valeur EA, d'une valeur de pondération correspondante, et dans lequel la génération de l'une des valeurs AHR de l'étape (d) implique la détermination d'une valeur de pondération normalisée pour chaque valeur de pondération d'une séquence des valeurs de pondération déterminées et implique en outre l'utilisation de chacune des valeurs de pondération normalisées pour pondérer sa valeur EHR correspondante dans une détermination de moyenne glissante pondérée de sorte qu'une sortie de la détermination de moyenne glissante pondérée est la valeur AHR.

3. Procédé selon la revendication 1, dans lequel la détermination dans l'étape (c) d'une valeur EA à partir d'un ensemble de données de sortie d'accéléromètre implique la détermination d'une valeur d'entropie spectrale de puissance à partir des valeurs de données de sortie de l'ensemble de données de sortie d'accéléromètre, ou dans lequel des périodes successives des périodes de temps dans l'étape (a) se chevauchent en partie les unes les autres dans le temps.

4. Procédé selon la revendication 1, comprenant en outre :
(e) l'affichage sur le téléphone intelligent d'une représentation d'au moins une des valeurs AHR générées dans l'étape (d).

5. Procédé selon la revendication 1, comprenant en outre :

(e) pour chaque AHR générée dans l'étape (d) la détermination si la valeur AHR présente ou non une caractéristique de précision ; et

(f) l'affichage sur le téléphone intelligent d'une représentation de chaque valeur AHR d'une séquence des valeurs AHR générées dans l'étape (d), dans lequel la représentation de chaque valeur AHR de la séquence est affichée conjointement avec une indication visuelle indiquant si la valeur AHR présente ou non la caractéristique de précision, dans lequel de préférence la représentation de chaque valeur AHR est un objet graphique rendu sur un écran du téléphone intelligent, et dans lequel l'indication visuelle indiquant si la valeur AHR présente ou non la caractéristique de précision est indiquée par une couleur de l'objet graphique.

6. Procédé selon la revendication 1, dans lequel les un ou plusieurs accéléromètres du téléphone intelligent comportent un accéléromètre d'axe x, un accéléromètre d'axe y et un accéléromètre d'axe z, et dans lequel chaque ensemble de données de sortie d'accéléromètre généré dans l'étape (a) comporte des valeurs de données de sortie d'accéléromètre qui sont générées par l'accéléromètre d'axe x, des valeurs de données de sortie d'accéléromètre qui sont générées par l'accéléromètre d'axe y, et des valeurs de données de sortie d'accéléromètre qui sont générées par l'accéléromètre d'axe z.

7. Procédé selon la revendication 1, comprenant en outre :

(e) le stockage d'informations de mise en correspondance de l'entropie spectrale et de la pondération sur le téléphone intelligent, et dans lequel la détermination de l'étape (c) implique la détermination d'une valeur PSE [power spectral entropy value - valeur d'entropie spectrale de puissance] pour chaque ensemble de données de sortie d'accéléromètre, puis pour chaque ensemble de données de sortie d'accéléromètre l'utilisation de la valeur PSE de l'ensemble de données de sortie d'accéléromètre et des informations stockées de mise en correspondance de l'entropie spectrale et de la pondération pour générer la valeur EA de l'ensemble de données de sortie d'accéléromètre.

8. Procédé comprenant :

(a) le stockage d'informations de mise en correspondance de l'entropie spectrale et de la pondération sur un téléphone intelligent ;
(b) l'utilisation d'un accéléromètre du téléphone intelligent pour générer un ensemble de données de sortie d'accéléromètre, dans lequel chaque ensemble de données de sortie d'accéléromètre contient une pluralité de valeurs de données de sortie d'accéléromètre pour une période de temps ;
(c) la détermination à partir de l'ensemble de données de sortie de l'accéléromètre d'une valeur EHR ;
(d) la détermination à partir de l'ensemble de données de sortie de l'accéléromètre d'une valeur PSE ; et
(e) l'utilisation de la valeur PSE déterminée dans l'étape (d) et des informations de mise en correspondance de l'entropie spectrale et de la pondération stockées dans l'étape (a) pour ajuster la valeur EHR, en générant ainsi une valeur AHR, dans lequel les étapes (a) à (e) sont effectuées par le téléphone intelligent.

9. Procédé selon la revendication 10, dans lequel les informations de mise en correspondance de l'entropie spectrale et de la pondération stockées dans l'étape (a) définissent une relation entre une version normalisée de la valeur PSE et une valeur EA, dans lequel la valeur EA détermine une valeur de pondération pour l'ensemble de données de sortie d'accéléromètre, et dans lequel l'étape (e) implique l'utilisation de la valeur de pondération pour l'ensemble de données de sortie d'accéléromètre pour ajuster la valeur EHR ou dans lequel les informations de mise en correspondance de l'entropie spectrale et de la pondération stockées dans l'étape (a) impliquent à la fois (1) une première mise en correspondance de l'entropie spectrale et de la pondération associée à un premier cas d'utilisation du téléphone intelligent et (2) une deuxième mise en correspondance de l'entropie spectrale et de la pondération associée à un deuxième cas d'utilisation du téléphone intelligent.

10. Procédé comprenant :

(a) le stockage de premières informations de mise en correspondance de l'entropie spectrale et de la pondération sur un téléphone intelligent ;
(b) le stockage de deuxièmes informations de mise en correspondance de l'entropie spectrale et de la pondération sur le téléphone intelligent ;
(c) l'utilisation d'un accéléromètre du téléphone intelligent pour générer un ensemble de données de sortie d'accéléromètre, dans lequel l'ensemble de données de sortie d'accéléromètre contient une pluralité de valeurs de données de sortie d'accéléromètre pour une période de temps ;
(d) la détermination à partir de l'ensemble de données de sortie de l'accéléromètre d'une valeur EHR ;
(e) la détermination à partir de l'ensemble de données de sortie de l'accéléromètre d'une valeur EBR [estimated breathing rate value - valeur de rythme respiratoire estimé] ;
(f) la détermination à partir des données de sortie de l'accéléromètre d'informations PSE ;
(g) l'utilisation des informations PSE déterminées dans l'étape (f) et des premières informations de mise en correspondance de l'entropie spectrale et de la pondération stockées dans l'étape (a) pour ajuster la valeur EHR, en générant ainsi une valeur AHR ; et
(h) l'utilisation des informations PSE déterminées dans l'étape (f) et des deuxièmes informations de mise en correspondance de l'entropie spectrale et de la pondération stockées dans l'étape (b) pour ajuster la valeur EBR, en générant ainsi une valeur ABR [adjusted breathing rate value - valeur de rythme respiratoire ajustée], dans lequel les étapes (a) à (h) sont effectuées par le téléphone intelligent.

11. Procédé selon la revendication 10, dans lequel l'étape (c) implique la génération d'une pluralité d'ensembles de données de sortie d'accélérateur, dans lequel l'étape (d) implique la détermination d'une valeur EHR pour chacun des ensembles de données de sortie d'accélérateur en déterminant ainsi une séquence de valeurs EHR, dans lequel l'étape (g) implique l'utilisation des premières informations de mise en correspondance de l'entropie spectrale et de la pondération pour générer une valeur de pondération pour chacune des valeurs EHR de sorte que chaque valeur EHR présente une valeur de pondération correspondante, et dans lequel la génération de la valeur AHR dans l'étape (g) implique la détermination d'une valeur moyenne glissante pondérée à partir d'une pluralité des valeurs EHR et de leurs valeurs de pondération correspondantes, dans lequel de préférence chaque ensemble de données de sortie d'accéléromètre contient une pluralité de valeurs de données de sortie d'accéléromètre pour une période de temps différente, et dans lequel des périodes de temps successives des périodes de temps différentes se chevauchent les unes les autres dans le temps.

12. Procédé selon la revendication 10, dans lequel une pluralité de mises en correspondance de l'entropie spectrale et de la pondération est stockée sur le téléphone intelligent, dans lequel les premières informations de mise en correspondance de l'entropie spectrale et de la pondération de l'étape (a) sont une des mises en correspondance de l'entropie spectrale et de la pondération de la pluralité de mises en correspondance de l'entropie spectrale et de la pondération, et dans lequel les deuxièmes informations de mise en correspondance de l'entropie spectrale et de la pondération dans l'étape (b) sont une autre des mises en correspondance de l'entropie spectrale et de la pondération de la pluralité de mises en correspondance de l'entropie spectrale et de la pondération.

13. Procédé selon la revendication 10, dans lequel le procédé comprend en outre :

(i) la détection par le téléphone intelligent d'un cas d'utilisation et, sur la base au moins en partie du cas d'utilisation détecté, la sélection par le téléphone intelligent de l'une des mises en correspondance de l'entropie spectrale et de la pondération à utiliser dans l'ajustement de la valeur EHR dans l'étape (g).

14. Procédé selon la revendication 10, dans lequel la valeur AHR générée dans l'étape (g) est une d'une séquence de valeurs AHR générées par le téléphone intelligent, dans lequel certaines des valeurs AHR de la séquence sont affichées graphiquement sur un écran du téléphone intelligent dans une première couleur, et dans lequel d'autres des valeurs AHR de la séquence sont affichées graphiquement sur l'écran du téléphone intelligent dans une deuxième couleur.

15. Procédé selon la revendication 10, dans lequel la valeur AHR générée dans l'étape (g) est une d'une séquence de valeurs AHR générées par le téléphone intelligent, dans lequel le téléphone intelligent détermine que certaines des valeurs AHR de la séquence sont relativement plus précises, tandis que le téléphone intelligent détermine que d'autres des valeurs AHR de la séquence sont relativement moins précises, dans lequel des valeurs AHR qui sont déterminées par le téléphone intelligent comme étant relativement plus précises sont affichées graphiquement sur un écran du téléphone intelligent dans une première couleur, et dans lequel des valeurs AHR qui sont déterminées par le téléphone intelligent comme étant relativement moins précises sont affichées graphiquement sur l'écran du téléphone intelligent dans une deuxième couleur ou dans lequel les informations PSE déterminées dans l'étape (f) comportent une première valeur PSE et une deuxième valeur PSE, dans lequel la première valeur PSE est utilisée dans la génération de la valeur AHR dans l'étape (g), et dans lequel la deuxième valeur PSE est utilisée dans la génération de la valeur ABR dans l'étape (h).

SMARTPHONE SOFTWARE STRUCTURE

# FIG. 1

100

START

STORE SPECTRAL ENTROPY TO WEIGHT MAPPING INFORMATION ON USER'S SMARTPHONE. ~101

IN RESPONSE TO USER ACTION, START HR AND BR APP ON SMARTPHONE. ~102

DISPLAY USER SELECTABLE OPTIONS INCLUDING A RELAXATION EXERCISE. ~103

DETECT USER SELECTION OF THE RELAXATION EXERCISE. ~104

DISPLAY INSTRUCTIONS TO USER ON HOW TO PERFORM THE EXERCISE - FOR EXAMPLE, INSTRUCT USER TO ASSUME A PROPER SITTING POSITION. ~105

BASED ON DETECTED USE PARAMETERS SELECT A CORRESPONDING SPECTRAL ENTROPY TO WEIGHT MAPPING FOR HEART RATE (SPMHR) AND A CORRESPONDING SPECTRAL ENTROPY TO WEIGHT MAPPING FOR BREATHING RATE (SPMBR). ~106

BUFFER CONSECUTIVE ACCELEROMETER OUTPUT DATA SAMPLES (X, Y, Z, AND TIME STAMP) FOR EACH OF A PLURALITY OF TIME PERIODS (FOR EXAMPLE, 1024 CONSECUTIVE ACCELEROMETER OUTPUT DATA SAMPLES PER EACH 20.48 SECOND OVERLAPPING TIME PERIOD) THEREBY OBTAINING A PLURALITY OF ACCELEROMETER OUTPUT DATA SETS. ~107

FOR EACH TIME PERIOD DETERMINE AN ESTIMATED HEART RATE (EHR) VALUE THEREBY DETERMINING A SEQUENCE OF EHR VALUES, AND FOR EACH TIME PERIOD DETERMINE AN ESTIMATED BREATHING RATE (EBR) VALUE THEREBY DETERMINING A SEQUENCE OF EBR VALUES. ~108

FROM EACH EHR VALUE AND THE SELECTED SPMHR DETERMINE A WEIGHT THEREBY DETERMINING A SEQUENCE OF EHR WEIGHTS, AND FROM EACH EBR VALUE AND THE SELECTED SPMBR DETERMINE A WEIGHT THEREBY DETERMINING A SEQUENCE OF EBR WEIGHTS. ~109

USE THE EHR WEIGHTS TO ADJUST EACH EHR VALUE THEREBY OBTAINING A SEQUENCE OF ADJUSTED HEART RATE (AHR) VALUES, AND USE THE EBR WEIGHTS TO ADJUST EACH EBR VALUE THEREBY OBTAINING A SEQUENCE OF ADJUSTED BREATHING RATE (ABR) VALUES. ~110

BASED ON AHR AND ABR VALUES SUPPLY CUSTOMIZED USER INFORMATION AND PROMPT TO THE USER (FOR EXAMPLE, AN INDICATION OF THE USER'S HR AND A BREATHING RHYTHM AUDIO PROMPT). ~111

112
RELAXATION EXERCISE COMPLETE          NO

YES

DISPLAY RESULTS OF THE RELAXATION EXERCISE ON SMARTPHONE DISPLAY. ~113

LOG RESULTS OF THE RELAXATION EXERCISE ON SMARTPHONE. ~114

END

FIG. 2

INSTRUCT USER TO ASSUME PROPER SITTING POSITION

## FIG. 3

USER IN PROPER SITTING POSITION

## FIG. 4

DISPLAY PROGRESS DURING RELAXATION EXERCISE

## FIG. 5

DISPLAY RESULTS OF RELAXATION EXERCISE

## FIG. 7

GRAPH PORTION
24

HEART RATE (BPM)

DOWNSAMPLED AHR VALUES
(ONE EVERY 10 SECONDS)

ECG REFERENCE
27

TIME (SECONDS)

630    820

GRAPH PORTION

FIG. 6

ACCURACY
(DIMENSIONLESS)

USE CASE = HR/SITTING POSITION/iOS.

FITTED
LINE
25

NORMALIZED SPECTRAL ENTROPY
(DIMENSIONLESS)

DETERMINATION OF A SPECTRAL
ENTROPY TO WEIGHT MAPPING

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10285650 B2
- US 10674424 B2
- US 10617308 B2
- EP 3318179 A2
- US 20140316191 A1
- WO 2006113900 A2
- US 2020260962 A1

**Non-patent literature cited in the description**

- **FEDERICA LANDREANI et al.** Beat-to-Beat Heart Rate Detection By Smartphone's Accelerometers: Validation With ECG. *Proceedings of the Annual International Conference of the IEEE Engineering in Medicine and Biology Society*, 2016
- **J. RAMOS-CASTRO et al.** Heart Rate Variability Analysis Using A Seismocardiogram Signal. *Conference Proceedings of the Annual International Conference of the IEEE Engineering in Medicine and Biology Society*, 2012, 5642-5645
- **LAHDENOJA OLLI et al.** Atrial Fibrillation Detection via Accelerometer and Gyroscope of a Smartphone. *IEEE Journal of Biomedical and Health Informatics*, January 2018